# EUROPÄISCHE PATENTSCHRIFT

(11) **EP 3 389 558 B1**
(45) Veröffentlichungstag und Bekanntmachung des Hinweises auf die Patenterteilung: **06.03.2024**
(21) Anmeldenummer: 16798659.5
(22) Anmeldetag: 09.11.2016
(51) Int. Cl.: A61F 2/08, A61B 17/00

(54) **BANDERHALTENDES IMPLANTAT**
LIGAMENT-PRESERVING IMPLANT
IMPLANT DE MAINTIEN POUR LIGAMENT

(30) Priorität: 15.12.2015 DE 102015225218
(43) Veröffentlichungstag der Anmeldung: 24.10.2018
(73) Patentinhaber: Kneesearch GmbH, 1723 Pierrafortscha (CH)
(72) Erfinder: EGGLI, Stefan Prof. Dr., 1723 Pierrafortscha (CH)
(74) Vertreter: Körfer, Thomas
(86) Internationale Anmeldenummer: PCT/EP2016/077150
(87) Internationale Veröffentlichungsnummer: WO 2017/102184

(56) Entgegenhaltungen:
- EP-A1- 1 493 404
- EP-A2- 0 051 954
- US-A- 3 613 120
- US-A1- 2007 162 121
- US-A1- 2007 185 532
- US-A1- 2009 054 928
- US-A1- 2009 157 193

## Beschreibung

### Technisches Gebiet

Die vorliegende Erfindung betrifft ein Implantat für den Einsatz in der Wiederherstellungschirurgie von Gelenken wie beispielsweise dem Kniegelenk bei einem Kreuzbandriss.

### Hintergrund und Aufgabe

Das menschliche Kniegelenk wird durch ein vorderes und ein hinteres Kreuzband stabilisiert. Bei einem starken Verdrehtrauma kann das Kreuzband entweder teilweise zerstört werden, wobei lediglich einige Fasern reißen, oder es kann zu einer kompletten Ruptur des Kreuzbands kommen. Dabei reißt das vordere Kreuzband 8 bis 10-mal häufiger als das hintere Kreuzband. Die operative Behandlung zur Heilung des Kreuzbands führt nur in weniger als der Hälfte der Behandlungen zu einem akzeptablen Resultat.

Eine zufriedenstellende Heilung, insbesondere bei einer kompletten Ruptur des vorderen Kreuzbands, ist relativ schwer zu erzielen, da die gerissenen Kreuzbandstümpfe sich anatomisch oft an falscher Stelle befinden und sich eine erforderliche primäre Heilungsmatrix am offenen Kniegelenk nicht bilden kann. Dies führt zu einer anhaltenden Instabilität des Kniegelenks, wodurch Folgeschäden bis hin zur Entwicklung von Arthrose auftreten können. Deshalb wird heutzutage bei einer kompletten Ruptur des vorderen Kreuzbands oft eine Entfernung der Kreuzbandstümpfe vorgenommen und das Kreuzband durch ein Sehnentransplantat ersetzt.

Die deutsche Offenlegungsschrift DE 38 03 208 A1 beschreibt beispielweise eine Vorrichtung für die Wiederherstellungschirurgie des Kniegelenks nach einem Kreuzbandriss. Dabei wird das Schienbein relativ zum Oberschenkelknochen stabilisiert, indem die Enden und die Winkellage eines Ersatzbandes im Kniegelenk an Knochenanbringungsplätzen durch eine chirurgische Vorrichtung präzise festgelegt werden. Der Nachteil dieser Vorrichtung und dem damit erzielten Resultat besteht darin, dass das vitale Gewebe des Kreuzbandes entfernt und durch ein avitales Ersatzband ersetzt wird. Dabei verliert das Kniegelenk die propriozeptive Führung, die durch ein vitales Band gewährleistet wird.

Das chinesische Gebrauchsmuster CN 203 953 876 U beschreibt ein streifenförmiges, hohlrohrartiges und faltbares künstliches Ligament aus gewobenen Material als Ersatz für ein vorderes Kreuzband, aufweisend mehrere unterschiedlich ausgeformte und aufeinanderfolgende Segmente. Durch die streifenförmige und faltbare Form soll die Kontaktfläche des künstlichen Bands mit dem Knochen verbreitert und die Befestigung am Knochen verbessert werden.

Die US Patentschrift US 5 702 422 A beschreibt ein Verfahren zur Reparatur eines Kreuzbandrisses des menschlichen Knies, wobei das gerissene Band innerhalb einer Spalte zwischen den beiden Konturen des Oberschenkelknochens mittels eines Bolzens und einer damit verbundenen Naht befestigt wird. Dabei soll die Heilung am Ansatz dadurch beschleunigt werden, dass in der Spongiosa des Knochens eine Vertiefung zum Auffangen von Blut ausgebildet ist. Der Nachteil dieses Verfahrens besteht darin, dass die Naht mit dem Bolzen verbunden ist und dadurch ein permanenter Zug auf das gerissene Band ausgeübt wird. Zusätzlich entsteht ein Trauma am Einbringungsort des Bolzens im Oberschenkelknochen.

Die US 2007/ 0 162 121 A1 beschreibt ein Verfahren und ein Mittel zur Reparatur und Rekonstruktion gerissener Bänder oder Sehnen. Das Mittel umfasst einen biologisch kompatiblen Gewebeklebstoff und eine biologisch abbaubare Faserfolie, ein Netz oder eine andere matrixartige Struktur, die zu einer Schutzhülle verarbeitet ist. Die Schutzhülse weist eine Flexibilität und Kontraktionsfähigkeit auf, die es erlauben, dass sich die Schutzhülse bei Ausdehnung der Schutzhülse in Längsrichtung zusammenzieht und so den Bereich der ausgefransten Ränder, der mit dem Gewebekleber behandelt wurde, komprimiert. Die Schutzhülse ist dabei so ausgelegt, dass sie einer Spannung standhält, die weitgehend der Spannung eines gesunden, funktionierenden Bands oder Sehne bei normaler körperlicher Aktivität entspricht. Die Schutzhülle wird bevor oder nachdem der Klebstoff vollständig auspolymerisiert ist über das behandelte Band gezogen. Alternativ wird, bevor oder nachdem das gerissene Band zusammengeklebt und der Klebstoff vollständig polymerisiert ist, der zusätzliche Gewebeklebstoff, noch in flüssiger Form, zugegeben, um die Schutzhülse zu füllen, und man lässt ihn polymerisieren, um die zusammengeklebten, gerissenen Ränder des Bandes oder der Sehne weiter zu stärken.

Die US 2009/ 0 157 193 A1beschreibt ein Verfahren und eine Vorrichtung zur Behandlung oder Heilung einer verletzten Sehne oder eines verletzten Bandes. Die Vorrichtung besteht im Wesentlichen aus einer Folie zur Sehnen- und Bänder-Reparatur. Diese Folie dient dazu, die zu reparierenden Bänder- oder Sehnen zusammenzuhalten, indem es mit Nähten auf der Sehne oder dem Band fixiert ist. Die Sehnen- und Bänderreparaturfolie hat eine poröse Schicht und eine dichtere Schicht und ein therapeutisches Mittel in der porösen Schicht, der dichteren Schicht oder in beiden. Die Reparaturfolie ist aus einem resorbierbaren oder nicht resorbierbaren Polymer hergestellt.

Die EP 1 493 404 A1beschreibt ein bioresorbierbares Bindegewebsgerüst zur Reparatur oder den Ersatz von Bändern und Sehnen. Das Bindegewebsgerüst enthält gegenüberliegende Verankerungssegmente die aus bioresorbierbaren Polymerfasern bestehen. Dabei sind die Polymerfasen parallel zu einer Längsachse des Gerüsts ausgerichtet. Weitere Polymerfasern sind quer zu einer Längsachse des Gerüsts ausgerichtet. Das Gerüst wird außerdem mit Gewebsfragmenten oder Zellen beaufschlagt, wodurch ein Nachwachsen und Ersetzten eines Bandes oder einer Sehne ermöglicht wird.

Die US 2009/ 054 928 A1beschreibt eine Vorrichtung zum Koppeln eines Weichgewebeimplantats in einem Knochen. Die Vorrichtung umfasst ein Element, um das Weichgewebeimplantat in einen Femurtunnel zu ziehen. Das Element besteht aus einem Nahtmaterial mit einem ersten und einem zweiten Ende, die durch eine erste und eine zweite Öffnung geführt werden, die dem Längskanal zugeordnet sind, um ein Paar von Schlaufen zu bilden. Abschnitte des Nahtmaterials liegen innerhalb der Naht parallel zueinander. Die Anwendung von Spannung auf die Nahtkonstruktion bewirkt die Retraktion des Weichteilimplantats in den Femurtunnel.

Die US 2007/ 185 532 A1beschreibt eine Anordnung zur Reparatur von Weichgewebe. Die Anordnung umfasst ein flexibles Element mit einem ersten und einem zweiten Ende und eine Litze, die durch das flexible Element verläuft. Die Litze erstreckt sich durch das flexible Element, so dass das Ziehen an mindestens einem der ersten und zweiten Litzenenden das flexible Element von einer ersten Form, die für das Einführen durch Weichgewebe geeignet ist, in eine zweite Form ändert, die für die sichere Unterbringung der Weichgewebereparaturanordnung relativ zum Weichgewebe geeignet ist.

Die EP 0 051 954 A2 zeigt eine Ersatzsehne oder ein Ersatzband, mit einer Vielzahl von Längssträngen aus einem biokompatiblen Gewebe. Die Enden der Stränge liegen in einem Gewebe und sind mit einem Film verbunden. Eine Hülse aus einem biokompatiblen Polymer umgibt die künstlichen Stränge, wobei die Stränge zueinander verschiebbar und in der Hülse beweglich sind. Dadurch ist die Last der Struktur gleichmäßig auf die Stränge verteilt. An den Enden der Struktur befinden sich Filme zum Fixieren der Struktur in der implantierten Position.

Die US 3 613 120 Abeschreibt künstliche Sehnen und insbesondere eine Prothese für Beugesehnen, die gewebte Endabschnitte enthält oder eine andere offene Maschenkonfiguration aufweisen, um das Einwachsen von fibrovaskulärem Gewebe zu fördern. Die künstliche Sehne enthält einen Mittelabschnitt, der aus mehreren parallelen Filamenten besteht, die dem Einwachsen von fibrovaskulärem Gewebe widerstehen und die freie Gleitbewegung des Mittelteils der Prothese fördern.

Der Erfindung liegt die Aufgabe zugrunde, eine Vorrichtung und ein Verfahren bereitzustellen, welche bzw. welches eine verbesserte Heilung bei einem Kreuzbandriss ermöglicht. Diese Aufgabe wird durch die unabhängigen Ansprüche gelöst. Die abhängigen Ansprüche stellen vorteilhafte Weiterbildungen des erfindungsgemäßen Gegenstands dar.

### Kurzbeschreibung der Erfindung

In einem ersten Aspekt betrifft die Erfindung ein Implantat für den Einsatz in ein Gelenk zur Stützung eines Ligaments, aufweisend einen zusammenhängenden Stützkörper, welcher einen inneren Hohlraum umschließt und welcher zur Führung eines Ligaments des Gelenks ausgebildet ist, und eine erste und zweite Öffnung in einer Außenwand des Stützkörpers, wobei die Außenwand des Stützkörpers diffusionshindernd ausgebildet ist. Die erste und zweite Öffnung sind an sich gegenüberliegenden Enden des Stützkörpers angeordnet und mit dem Hohlraum des Stützkörpers verbunden.

Das erfindungsgemäße Implantat ermöglicht durch diffusionshindernde Außenwand die Ausbildung oder Bereitstellung einer primären Heilungsmatrix wie beispielsweise einem primären Blutkoagel in unmittelbarer Umgebung des im Hohlraum geführten Ligaments. Hierdurch wird neben einer stabilisierenden Führung bzw. Stützung eines wiederhergestellten bzw. repositionierten Ligaments ein deutlich verbesserter Heilungsverlauf gegenüber den bekannten Wiederherstellungsverfahren ermöglicht.

Die diffusionshindernde Außenwand des Stützkörpers ist erfindungsgemäß derart ausgebildet, dass ein Flüssigkeitstransport vom Hohlraum des Stützkörpers hin zur Umgebung verhindert wird. Hierbei wird Flüssigkeit an einem Verlassen des Hohlraums durch die Außenwand gehindert. Die diffusionshindernde Außenwand kann beispielsweise durch eine im Wesentlichen geschlossene Oberfläche gebildet sein. Die Oberfläche kann jedoch auch Poren oder Mikroporen aufweisen.

Das Implantat ist vorzugsweise derart ausgebildet, um eine seitliche Führung des Ligaments zu ermöglichen. Hierdurch werden eine einfachere Adaptation der gerissenen Ligamentfasern und eine seitliche Stabilisierung des wiederhergestellten bzw. repositionierten Ligaments erzielt.

Der Hohlraum des Stützkörpers ist erfindungsgemäß derart dimensioniert, dass die Ausbildung einer primären Heilungsmatrix, wie beispielsweise einem primären Blutkoagel, um ein im Hohlraum geführtes Ligament ermöglicht wird. Ein Innendurchmesser des Hohlraums ist hierbei an die seitliche Erstreckung des Kreuzbandes angepasst und eine Innenwand des Hohlraums umschließt das zu führende Ligament am Umfang enganliegend.

In den inneren Hohlraum des Stützkörpers sind erfindungsgemäß ein oder mehrere Wachstumsfaktoren und/oder die Heilung fördernde Substanzen durch ein Injektionsmittel injizierbar.

Der Stützkörper besteht vorzugsweise wenigstens teilweise aus elastischem Material. Der Stützkörper kann vorzugsweise in Längsrichtung und/oder lateraler Richtung elastisch gestreckt bzw. gedehnt werden. Hierdurch kann sich der Stützkörper an den Umfang bzw. die Form und Länge des zu stützenden Ligaments individuell anpassen. Des Weiteren ist die Ausbildung einer primären Heilungsmatrix in unmittelbarer Nähe zum Ligament durch das elastische Material möglich, ohne dass die Gefahr eines Aufreißens des Stützkörpers besteht.

Der Stützkörper ist vorzugsweise im Wesentlichen schlauchförmig oder ballonförmig ausgeprägt. Die seitliche Erstreckung bzw. die Breite des Stützkörpers kann dabei homogen entlang der länglichen Erstreckungsrichtung des Stützkörpers sein. Der Stützkörper kann jedoch auch andere geometrische Formen aufweisen, welche ein Stützung bzw. Führung des Ligaments vorzugsweise in Längsrichtung des Ligaments ermöglichen.

Der Stützkörper kann eine netzförmige, schaumartige oder gelartige Struktur aufweisen. Beispielsweise kann der Stützkörper aus einem engmaschig gewobenen Material bestehen. Die Poren des Materials können hierbei als Diffusionsbarriere ausgeprägt sein.

Der Stützkörper besteht vorzugsweise aus einem vom menschlichen Körper resorbierbarem Material. Der Stützkörper kann jedoch auch aus einem nicht resorbierbaren Material bestehen. Das Material des Stützkörpers kann eines oder mehrere der folgenden Materialien umfassen: Collagen Typ 1 oder 2, Polyurethan und/oder Polyclycolsäure. Der Stützkörper ist vorzugsweise vollständig aus einem der oben genannten Materialien geformt. Insbesondere die Außenwand ist vorzugsweise integral mit dem Stützkörper ausgebildet.

In einem bevorzugten Ausführungsbeispiel weist das Implantat wenigstens teilweise eine für ein Ligament wachstumsfördernde Substanz auf. Diese kann beispielsweise auf der Außenwand angeordnet oder in diese eingebracht sein. Vorzugsweise ist eine Innenfläche der Außenwand des Stützkörpers mit der wachstumsfördernden Substanz wenigstens teilweise beschichtet.

Die wachstumsfördernde Substanz kann eine oder mehrere der folgenden Substanzen umfassen: pluripotente Stammzellen, Fibrin, extrazelluläre Matrixproteine, Vitronektin, Stammzellen aus Eigenfett, Wachstumskatore, Zytokine, HGF (Human Growth Factor), Interleukin-1, IL-3, IL-6, IL-11, PDGF (platelet derived growth factor), FGF (fibroblast growth factor), EGF (epidermal growth factor), VEGF (vascular endothelial growth factor), PRP (platelet rich plasma), PRF (plateler rich fibrin) und/oder Angiopoetine.

Das Implantat kann an den beiden gegenüberliegenden Enden Befestigungsmittel zur Befestigung und Positionierung an einen Knochen aufweisen. Diese können integral mit dem Stützkörper ausgebildet sein. Die Befestigungsmittel können beispielsweise Ösen umfassen, an welche eine Verbindungsnaht zwischen Implantat und einem Knochen befestigt werden kann. Hierdurch kann das Implantat bezüglich eines Knochens und dem im Implantat geführten Ligament fix positioniert werden. Die hierfür verwendete Naht kann aus einem vom menschlichen Körper resorbierbarem Material bestehen, beispielsweise PDS (Polydioxanon) oder PGS (PolyGlycolsäure). In einem bevorzugten Ausführungsbeispiel können auch eine oder mehrere derartige Befestigungsnähte integral mit dem Implantat geformt sein.

Das Implantat ist vorzugsweise für den Einsatz in einem menschlichen Kniegelenk vorgesehen. Dabei ist das Implantat derart ausgebildet, um eine Führung eines Ligaments des Kniegelenks, insbesondere des vorderen Kreuzbandes zu ermöglichen. Das Implantat kann auch zur Führung von anderen Ligamenten des menschlichen oder tierischen Körpers verwendet werden.

In einem nicht zur Erfindung gehörenden Beispiel wird ein Verfahren zur Wiederherstellung eines gerissenen Bandes, insbesondere Kreuzbandes, in einem menschlichen oder tierischen Gelenk, insbesondere Kniegelenk, mit Hilfe eines Implantats beschrieben, aufweisend die Schritte:
- Fassen der Stümpfe eines gerissenen Teils des Bandes mittels einer Naht,
- Ummantelung des gerissenen Teils des Bandes mit einem Implantat aufweisend einen Hohlraum umschließenden Stützkörper, so dass der gerissenen Teil des Bandes in dem Hohlraum des Stützkörpers geführt wird, wobei eine Außenwand des Stützkörpers wenigstens teilweise diffusionshindernd ausgebildet ist,
- anatomische Reposition des gerissenen Teils des Bandes an einen Knochen des Gelenks und/oder an das entsprechende Gegenstück des gerissenen Bandes,
- Fixation des Implantats an einen Knochen des Gelenks, wie beispielsweise dem Oberschenkel und/oder Schienbeinknochen.

Durch das nicht zur Erfindung gehörende Verfahren wird eine einfache und effiziente Wiederherstellung des gerissenen Bandes, insbesondere Kreuzbandes, ermöglicht, wobei die Ummantelung des Bandes mit Hilfe eines hohlraumaufweisenden und diffusionshindernden Implantats eine verbesserte Heilung des repositionierten Kreuzbandes ermöglicht. Hierbei wird insbesondere die Bildung einer primären Heilungsmatrix vereinfacht bzw. ermöglicht, welche sich entlang des repositionierten Kreuzbandes innerhalb des Hohlraums des Implantats ausbilden kann. Obwohl sich das Verfahren auch für andere Bänder eignet, wird es im Folgenden anhand des Kreuzbandes erläutert.

Bei der Ausrichtung und/oder Adaptation der gerissenen Stümpfe des Kreuzbandes werden diese zunächst mit einer Naht gefasst. Hierbei können eine, zwei oder mehrere Nähte durch die Stümpfe des Kreuzbandes geführt werden. Danach erfolgt die Ummantelung der Kreuzbandstümpfe mit Hilfe des Implantats. Dieses kann beispielsweise durch die zuvor bereitgestellten Nähte hin zu den Kreuzbandstümpfen geführt und auf diese geschoben bzw. über diese gestülpt werden. Hierdurch erfolgt eine Adaptation der aufgefaserten Kreuzbandstümpfe. Des Weiteren wird ein die Kreuzbandstümpfe umgebener Hohlraum im Inneren des Stützkörpers generiert, in welchem sich eine primäre Heilungsmatrix wie beispielsweise ein primäres Blutkoagel bilden kann. Ein Innendurchmesser des Implantathohlraums ist hierbei vorzugsweise an den Außendurchmesser bzw. die seitliche Erstreckung des Kreuzbandes angepasst. Zusätzlich erfolgt durch die Aufbringung des Implantats auf die Kreuzbandstümpfe eine seitliche Schienung des gerissenen Kreuzbands.

Das Fassen der Kreuzbandstümpfe, die Adaptation der Stümpfe und die Ummantelung des gerissenen Teils des Kreuzbands erfolgt vorzugsweise mit Hilfe eines arthroskopischen Applikators.

Das Verfahren kann ferner eine Injektion von zusätzlichen Wachstumsfaktoren oder heilungsfördernden Substanzen in den Hohlraum des Stützkörpers umfassen. Dies kann mit Hilfe eines geeigneten Injektionsmittels erfolgen, mit welchem beispielsweise die Außenwand des Implantats wenigstens an einer Stelle zur Injektion perforiert werden kann.

Die Wachstumsfaktoren oder heilungsfördernden Substanzen können eine oder mehrere der folgenden Substanzen umfassen: Frischblut zur Gerinnung im Stützkörper, Blut aus dem Markraum mit pluripotenten Stammzellen, Fibrin, extrazelluläre Matrixproteine, Vitronektin, Stammzellen aus Eigenfett, Wachstumskatore, Zytokine, HGF (Human Growth Factor), Interleukin-1, IL-3, IL-6, IL-11, PDGF (platelet derived growth factor), FGF (fibroblast growth factor), EGF (epidermal growth factor), VEGF (vascular endothelial growth factor), PRP (platelet rich plasma), PRF (plateler rich fibrin), und/oder Angiopoetine.

Das Verfahren weist vorzugsweise einen Schritt zur intraartikulären Stabilisation des Implantats auf. Dies kann beispielsweise durch eine Zusatznaht, autoelastische Spannung und/oder durch zusätzliche Fixation des Implantats an einen Knochen erfolgen. Hierbei können zur Befestigung des Implantats entweder zusätzliche Nähte am Implantat, zwischen Implantat und Kreuzband oder zwischen Implantat und Knochen angebracht werden. Die jeweilige Naht kann an entsprechend vorgeformten Befestigungsmitteln wie beispielsweise Ösen am Implantat befestigt werden. Knochenseitig kann die Befestigungsnaht beispielweise an eingebrachten Bolzen befestigt werden. Das Implantat kann sich zusätzlich oder alternativ durch dessen Eigenelastizität stabilisieren.

Vorzugsweise weist das Verfahren weiterhin den Schritt einer intra- oder extraartikulären Stabilisation des Kniegelenks auf. Eine derartige Stabilisation kann beispielsweise mittels einer extraartikulären Schiene erfolgen. Alternativ oder zusätzlich kann eine dynamische intraligamentäre Stabilisation (Ligamys ^{™}) und/oder eine intraartikuläre Punkt-zu-Punkt-Verbindung (Internal Brace^{™}) zur Stabilisation verwendet werden.

Das Implantat und die beim Verfahren verwendeten Nähte sind vorzugsweise aus einem vom menschlichen oder tierischen Körper resorbierbarem Material hergestellt. Das Implantat muss somit nicht sekundär durch eine zusätzliche Operation entfernt werden. Das Implantat ist vorzugsweise aus Collagen Typ 1 oder 2, Polyurethan und/oder Polyclycolsäure geformt. Die Naht bzw. die beim Verfahren verwendeten Nähte sind vorzugsweise aus PDS (Polydioxanon) und/oder PGS (PolyGlycolsäure) geformt.

Das gesamte Verfahren kann arthroskopisch durchgeführt werden, das heisst ohne zusätzliche Inzisionen im Vergleich zur herkömmlichen Arthroskopie anzubringen.

Das für das Verfahren verwendete Implantat kann einen im Wesentlichen rohr- oder ballonförmigen Stützkörper aufweisen. Das Implantat kann eine netzförmige Struktur, eine schaumartige oder gelartige Struktur aufweisen. Das Implantat kann vorzugsweise wenigstens teilweise elastisch ausgeprägt sein. Das für das Verfahren verwendete Implantat kann des Weiteren wie oben in Bezug auf die Erfindung beschrieben ausgebildet sein.

### Kurzbeschreibung der Figuren

Nachfolgend wird ein Ausführungsbeispiel der Erfindung lediglich beispielhaft beschrieben und eine detaillierte Beschreibung der Figuren der Zeichnung gegeben.
- Fig. 1: ist eine schematische Zeichnung eines bevorzugten Ausführungsbeispiels des erfindungsgemäßen Implantats;
- Fig. 2: ist eine schematische Zeichnung eines menschlichen Kniegelenks mit einem proximal gerissenen Kreuzband zwischen Oberschenkel- und Schienbeinknochen;
- Fig. 3: ist eine schematische Ansicht des Kniegelenks gemäß Fig. 2 und zeigt das Fassen der ausgefransten Kreuzbandstümpfe mittels einer Naht;
- Fig. 4: ist eine schematische Ansicht des Kniegelenks und zeigt das Einbringen eines bevorzugten Ausführungsbeispiels des erfindungsgemäßen Implantats auf das proximal gerissene Kreuzband;
- Fig. 5: ist eine schematische Ansicht des Kniegelenks und zeigt das Überstülpen eines bevorzugten Ausführungsbeispiels des erfindungsgemäßen Implantats auf das proximal gerissene Kreuzband;
- Fig. 6: ist eine schematische Ansicht des Kniegelenks und zeigt die anatomische Reposition des proximal gerissenen Kreuzbandes an die Abrissstelle des Oberschenkelknochens und
- Fig. 7: ist eine schematische Ansicht des Kniegelenks und zeigt die Fixation des Implantats und die Injektion von heilungsfördernden Substanzen.

### Detaillierte Beschreibung der Ausführungsbeispiele

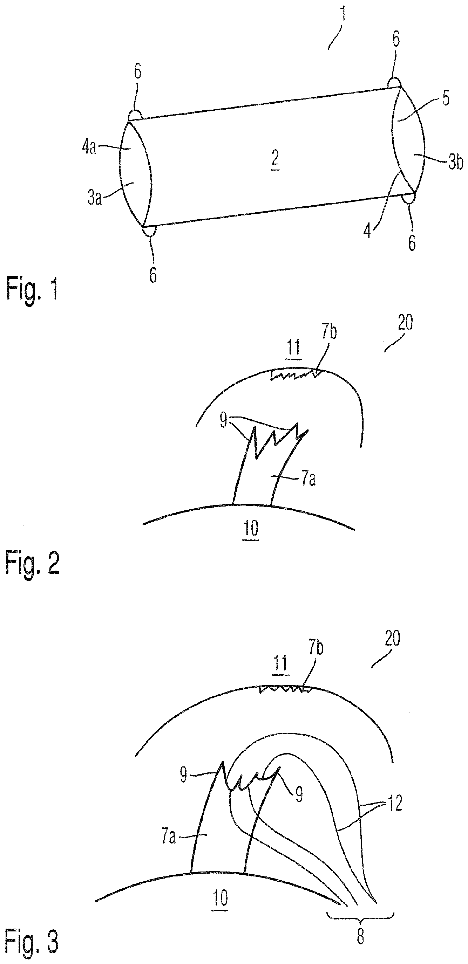

Fig. 1 zeigt ein bevorzugtes Ausführungsbeispiel des erfindungsgemäßen Implantats 1 für den Einsatz in ein Gelenk zur Stützung eines Ligaments, beispielsweise dem vorderen Kreuzband eines menschlichen Kniegelenks.

Das Implantat 1 weist einen zusammenhängenden Stützkörper 2 auf, welcher im vorliegenden Ausführungsbeispiel im Wesentlichen rohr- bzw. schlauchförmig ausgebildet ist. Der Stützkörper kann jedoch auch eine andere geometrische Form aufweisen.

Der Stützkörper weist einen inneren vorzugsweise zylinderförmigen Hohlraum 5 auf, welcher von einer Außenwand 4 umschlossen ist. An gegenüberliegenden Enden des Stützkörpers sind Öffnungen 3a,3b in der Außenwand 4 vorgesehen. Ein zu stützendes oder zu führendes Ligament kann somit durch die Öffnungen 3a,3b und durch den inneren Hohlraum 5 geführt werden.

Die Außenwand 4 des Stützkörpers 2 ist diffusionshindernd ausgebildet, vorzugsweise derart, um einen Flüssigkeitstransport vom Hohlraum 5 des Stützkörpers hin zur Umgebung des Implantats 1 stark einzuschränken oder zu verhindern. Hierdurch wird die Bildung einer primären Heilungsmatrix im Inneren des Hohlraums und in unmittelbarer Nähe zum geführten Ligament ermöglicht.

Der Stützkörper 2 kann eine netzförmige, schaumartige oder gelartige Struktur aufweisen. Der Stützkörper kann beispielsweise aus einem engmaschig gewobenen Material bestehen.

Der Hohlraum 5 ist vorzugsweise bezüglich dessen innerer Abmessungen derart ausgeprägt, dass eine Innenwand 4a des Hohlraums 5 ein zu führendes Ligament seitlich eng umschließt. Der Stützkörper 2 kann wenigstens teilweise aus elastischem Material bestehen. Dies gewährleistet eine optimale Anpassung des Stützkörpers 2 an die Außenkonturen des zu führenden Ligaments.

Das Implantat 1 kann optional Befestigungsmittel 6 zur Befestigung und Positionierung des Implantats an einen Knochen aufweisen. Diese können durch Ösen 6 gebildet sein, welche an gegenüberliegenden Enden des Stützkörpers angebracht oder integral mit dem Stützkörper geformt sind.

Der Stützkörper 2 besteht vorzugsweise aus einem vom menschlichen Körper resorbierbaren Material. Das Material des Stützkörpers 2 kann beispielsweise Collagen Typ 1 oder 2, Polyurethan und/oder Polyclycolsäure umfassen.

Der Stützkörper 2 kann wenigstens teilweise eine für ein Ligament wachstumsfördernde Substanz aufweisen. Diese kann in das Material der Außenwand des Stützkörpers eingebracht sein und/oder auf die Innenfläche 4a der Außenwand aufgebracht sein, welche den Hohlraum 5 begrenzt.

Das nicht zur Erfindung gehörende Verfahren wird im Folgenden in Bezug auf die Fig. 2 bis 7 beschrieben.

Fig. 2 ist eine schematische Darstellung eines menschlichen Kniegelenks 20 und zeigt ein proximal gerissenes Kreuzband 7a,7b zwischen Oberschenkel- und Schienbeinknochen 10,11. Das untere gerissene Teilstück 7a des Kreuzbandes ist am Schienbeinknochen 10 befestigt. Das Gegenstück 7b des Kreuzbandes ist am Oberschenkelknochen 11 angebracht. Dieses Gegenstück 7b stellt einen femoralen "Footprint" des Kreuzbandes dar, an welchem das proximal gerissene Teilstück 7a des Kreuzbandes zu befestigen ist.

Wie in Fig. 3 gezeigt, werden hierbei zunächst die Stümpfe bzw. gerissenen Faserenden 9 des Kreuzbandstücks 7a mit Hilfe einer oder mehrerer ins Kniegelenk 20 eingebrachten Nähte 12 gefasst. Die Naht bzw. die Nähte 12 werden hierbei durch die Faserenden 9 gestochen.

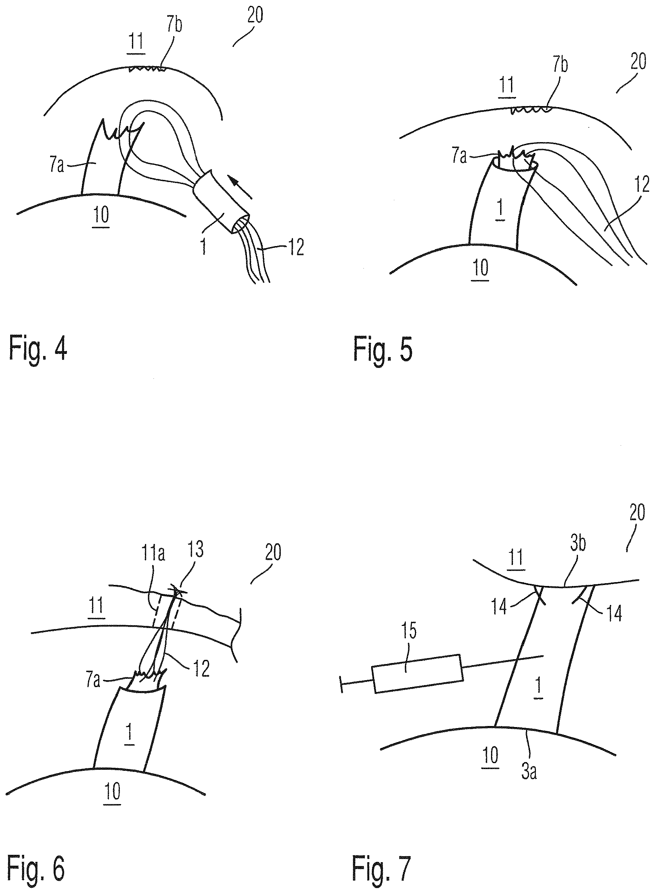

Danach wird das Implantat 1 mit Hilfe der wenigstens einen Naht 12 auf das Kreuzbandteilstück 7a aufgebracht. Dies kann beispielsweise dadurch erfolgen, dass die jeweiligen Enden der Nähte 12 zu einer Schlaufe 8 geformt werden, über welche das Implantat 1 mittels der Öffnungen 3a,3b hin zur den Faserenden 9 geschoben und über das Kreuzbandteilstück 7a gestülpt werden kann (siehe Fig. 4 und 5).

Das Implantat 1 wird hierbei derart positioniert, dass das Kreuzbandteilstück 7a innerhalb des Hohlraums 5 des vorzugsweise schlauchförmigen Stützköpers 2 geführt wird. Die Innenwand 4a des Stützkörpers liegt hierbei vorzugsweise eng am Umfang des Kreuzbandteilstücks 7a an. Es wird demnach eine Schienung des Kreuzbandteilstücks 7a mit Hilfe des aufgesetzten Implantats 1 als auch eine Ausrichtung bzw. Adaptation der Kreuzbandstümpfe 9 erzielt. Durch das Eigengewicht des Implantats 1 liegt dieses auf dem Schienbeinknochen 10 auf.

Das Fassen der Kreuzbandstümpfe 9 und die Ummantelung des gerissenen Kreuzbandteilstücks 7a erfolgt vorzugsweise mit Hilfe eines arthroskopischen Applikators.

Anschließend erfolgt die anatomische Reposition des gerissenen Kreuzbandteilstücks 7a an den femoralen "Footprint" 7b des Kreuzbandes am Oberschenkelknochen 11, wie in Fig. 6 gezeigt. Hierbei wird das gerissene Teilstück 7a bzw. dessen Faserenden 9 mit Hilfe der Naht bzw. Nähte 12 zum Oberschenkelknochen 11 gedehnt und an diesem befestigt. Dies kann beispielsweise mit Hilfe eines im Knochen 11 vorgesehenen Befestigungslochs 11a erfolgen, welches zuvor in den Knochen zu bohren ist. Darin kann die Naht bzw. die Nähte 12 mit Hilfe von konventionellen Verbindungstechniken wie beispielsweise Knüpfen der Nahtenden 13 fixiert werden. Alternativ oder zusätzlich zum Vorsehen eines Bohrlochs 11a im Knochen 11 kann ein Anker bzw. Bolzen (nicht gezeigt) in den Knochen eingebracht werden, um die Naht 12 daran zu fixieren.

Nach der Reposition des gerissenen Kreuzbands 7a,7b kann sich das Implantat zwischen Oberschenkelknochen 11 und Schienbeinknochen 10 beispielsweise durch dessen Eigenelastizität selbst positionieren. Hierbei liegen vorzugsweise die Öffnungen 3a,3b des Implantats 1 am Oberschenkelknochen 11 bzw. am Schienbeinknochen 10 an. Die Innenwand 4a des Implantats 1 liegt eng am wiederhergestellten Kreuzband 7a,7b an, wodurch eine Führung und Stützung des Kreuzbandes 7a,7b erzielt wird. Durch die diffusionshindernde Außenwand 4 des Implantats wird die lokale Ausbildung einer primären Heilungsmatrix im Inneren des Implantats und entlang des wiederhergestellten Kreuzbands 7a,7b ermöglicht.

Optional können durch Injektion beispielsweise mittels einer Spritze 15 zusätzliche Wachstumsfaktoren oder heilungsfördernden Substanzen in den Hohlraum 5 des Implantats 1 eingebracht werden. Diese können die folgenden Substanzen umfassen: Frischblut zur Gerinnung im Stützkörper 2, Blut aus dem Markraum mit pluripotenten Stammzellen, Fibrin, extrazelluläre Matrixproteine, Vitronektin, Stammzellen aus Eigenfett, Wachstumskatore, Zytokine, HGF (Human Growth Factor), Interleukin-1, IL-3, IL-6, IL-11, PDGF (platelet derived growth factor), FGF (fibroblast growth factor), EGF (epidermal growth factor), VEGF (vascular endothelial growth factor), PRP (platelet rich plasma), PRF (plateler rich fibrin), und/oder Angiopoetine.

Durch die diffusionshindernde Außenwand 4 wird die eingebrachte Substanz innerhalb des Hohlraums 5 gehalten. Dies ist insbesondere bei eingebrachten Stammzellen vorteilhaft, da diese negative Auswirkungen wie beispielsweise ungewünschten Knorpelwachstum begünstigen können, wenn diese an eine andere Stelle des Kniegelenks oder des menschlichen Körpers transportiert werden. Alternativ zu der beschriebenen Einbringung von Wachstumsfaktoren oder heilungsfördernden Substanzen in das Implantat 1 kann die Innenwand 4a des Implantats mit einer oder mehreren der oben genannten Substanzen beschichtet sein.

Zur weiteren Stabilisierung des Implantats 1 im Kniegelenk können zusätzliche Fixiernähte 14 vorgesehen werden, welche zwischen Implantat 1 und einem oder beiden der Knochen 10,11 angebracht werden. Die Fixiernähte 14 können beispielsweise an optionalen Befestigungsmitteln 6 des Implantats (siehe Fig. 1) befestigt werden. An den Knochen 10, 11 können die Fixiernähte 14 mit bekannten Verfahren befestigt werden. Beispielsweise können Bohrlöcher und/oder Bolzen in den jeweiligen Knochen 10,11 zur Befestigung der Nähte 14 vorgesehen werden. Im fixierten Zustand des Implantats 1 wie in Fig. 7 gezeigt liegen die Öffnungen 3a,3b vorzugsweise direkt am Oberschenkel- bzw. Schienbeinknochen 10,11 an.

Die Erfindung ist nicht auf die dargestellten Ausführungsbeispiele beschränkt. Sie eignet sich nicht nur zur Behandlung des Kreuzbandes, sondern auch zur Behandlung anderer Bänder des Kniegelenks und anderer Gelenke, wie beispielsweise des Ellbogengelenks oder des Schultergelenks. Es eignet sich auch nicht nur für den menschlichen Körper sondern auch für den tierischen Körper.

## Patentansprüche

1. Implantat (1) für den Einsatz in ein Gelenk (20) zur Stützung eines Ligaments (7a,7b), aufweisend
einen zusammenhängenden Stützkörper (2), welcher einen inneren Hohlraum (5) umschließt und welcher zur Führung eines Ligaments (7a,7b) des Gelenks (20) ausgebildet ist, und
eine erste und zweite Öffnung (3a,3b) in einer Außenwand (4) des Stützkörpers,
wobei die Außenwand (4) des Stützkörpers (2) diffusionshindernd ausgebildet ist, so dass ein Flüssigkeitstransport vom inneren Hohlraum (5) hin zur Umgebung des Implantats (1) verhindert ist,
**dadurch gekennzeichnet,**
**dass** der Innendurchmesser des Hohlraums (5) des Stützkörpers (2) an die seitliche Erstreckung des Ligaments (7a, 7b) angepasst ist und eine Innenwand (4a) des Hohlraums (5) ein zu führendes Ligament (7a,7b) am Umfang enganliegend umschließt, so dass die Ausbildung einer primären Heilungsmatrix um das im Hohlraum geführte Ligament (7a,7b) ermöglicht ist, und
**dass** in den inneren Hohlraum (5) ein oder mehrere Wachstumsfaktoren und/oder die Heilung fördernde Substanzen durch ein Injektionsmittel (15) injizierbar sind, und
**dass** die erste und zweite Öffnung (3a,3b) an sich gegenüberliegenden Enden des Stützkörpers (2) angeordnet und mit dem Hohlraum (5) des Stützkörpers verbunden sind.

2. Das Implantat gemäß Anspruch 1,
wobei der Stützkörper (2) wenigstens teilweise aus elastischem Material besteht.

3. Das Implantat gemäß Anspruch 1 oder 2,
wobei der Stützkörper (2) im Wesentlichen schlauchförmig oder ballonförmig ist.

4. Das Implantat gemäß einem der Ansprüche 1 bis 3,
wobei der Stützkörper (2) eine netzförmige, schaumartige oder gelartige Struktur aufweist.

5. Das Implantat gemäß einem der vorhergehenden Ansprüche,
wobei der Stützkörper (2) aus einem engmaschig gewobenen Material besteht.

6. Das Implantat gemäß einem der vorherigen Ansprüche,
wobei der Stützkörper (2) aus einem vom menschlichen oder tierischen Körper resorbierbarem Material besteht.

7. Das Implantat gemäß einem der vorherigen Ansprüche,
wobei das Material des Stützkörpers (2) eines oder mehrere der folgenden Materialien umfasst: Collagen Typ 1 oder 2, Polyurethan, Polyclycolsäure.

8. Das Implantat gemäß einem der vorherigen Ansprüche,
wobei der Stützkörper (2) wenigstens teilweise eine für ein Ligament (7a,7b) wachstumsfördernde Substanz aufweist.

9. Das Implantat gemäß Anspruch 8,
wobei eine Innenfläche (4a) der Außenwand (4) des Stützkörpers (2) mit der wachstumsfördernden Substanz wenigstens teilweise beschichtet ist.

10. Das Implantat gemäß Anspruch 8 oder 9,
wobei die wachstumsfördernde Substanz eine oder mehrere der folgenden Substanzen umfasst: pluripotente Stammzellen, Fibrin, extrazelluläre Matrixproteine, Vitronektin, Stammzellen aus Eigenfett, Wachstumskatore, Zytokine, HGF (Human Growth Factor), Interleukin-1, IL-3, IL-6, IL-11, PDGF (platelet derived growth factor), FGF (fibroblast growth factor), EGF (epidermal growth factor), VEGF (vascular endothelial growth factor), PRP (platelet rich plasma), PRF (plateler rich fibrin) und/oder Angiopoetine.

11. Das Implantat gemäß einem der vorherigen Ansprüche,
wobei der Stützkörper (2) an den beiden gegenüberliegenden Enden Befestigungsmittel (6) zur Befestigung und Positionierung an einen Knochen (10,11) aufweist.

## Claims

1. An implant (1) for use in a joint (20) for the support of a ligament (7a, 7b), comprising a coherent support element (2), which encloses an internal cavity (5), and which is embodied for the guiding of a ligament (7a, 7b) of the joint (20), and
a first and second opening (3a, 3b) in an outer wall (4) of the support element,
wherein the outer wall (4) of the support element (2) is embodied in a diffusion-inhibiting manner, so that a fluid transport from the internal cavity (5) towards the environment of the implant (1) is prevented,
**characterised in that,**
the internal diameter of the cavity (5) of the support element (2) is adapted to the lateral extension of the ligament (7a, 7b) and an inner wall (4a) of the cavity (5) tightly encloses the periphery of a ligament (7a, 7b) to be guided, so that the formation of a primary healing matrix around the ligament (7a, 7b) guided in the cavity is made possible, and
that one or more growth factors and/or healing-promoting substances can be injected into the internal cavity (5) via an injection means (15), and
that the first and second opening (3a, 3b) are arranged at opposite ends of the support element (2) and are connected to the cavity (5) of the support element.

2. The implant according to claim 1,
wherein the support element (2) consists at least partially of elastic material.

3. The implant according to claim 1 or 2,
wherein the support element (2) is substantially tubular or balloon-shaped.

4. The implant according to any one of claims 1 to 3,
wherein the support element (2) comprises a reticular, foam-like or gel-like structure.

5. The implant according to any one of the preceding claims,
wherein the support element (2) consists of a fine-meshed woven material.

6. The implant according to any one of the preceding claims,
wherein the support element (2) consists of a material resorbable by the human or animal body.

7. The implant according to any one of the preceding claims,
wherein the material of the support element (2) comprises one or more of the following materials: collagen type 1 or 2, polyurethane, polyglycolic acid.

8. The implant according to any one of the preceding claims,
wherein the support element (2) comprises at least partially a substance which is growth-promoting for a ligament (7a, 7b).

9. The implant according to claim 8,
wherein an inner face (4a) of the outer wall (4) of the support element (2) is at least partially coated with the growth-promoting substance.

10. The implant according to claim 8 or 9,
wherein the growth-promoting substance comprises one or more of the following substances: pluripotent stem cells, fibrin, extracellular matrix proteins, vitronectin, stem cells from autologous fat, growth factors, cytokine, HGF (Human Growth Factor), interleukin-1, IL-3, IL-6, IL-11, PDGF (platelet derived growth factor), FGF (fibroblast growth factor), EGF (epidermal growth factor), VEGF (vascular endothelial growth factor), PRP (platelet rich plasma), PRF (platelet rich fibrin) and/or angiopoetins.

11. The implant according to any one of the preceding claims,
wherein the support element (2) comprises attachment means (6) at both opposing ends for attachment and positioning on a bone (10, 11).

## Revendications

1. Un implant (1) destiné à être utilisé dans une articulation (20) pour soutenir un ligament (7a, 7b), comportant
un corps de soutien cohésif (2) qui comprend une cavité intérieure (5) et qui est conçu pour guider un ligament (7a, 7b) de l'articulation (20), et
une première et une seconde ouverture (3a, 3b) dans une paroi extérieure (4) du corps de soutien,
dans lequel la paroi extérieure (4) du corps de soutien (2) est conçue pour empêcher la diffusion, de sorte qu'un transport de liquide est empêché de la cavité intérieure (5) vers l'environnement de l'implant (1),
**caractérisé en ce que**
le diamètre intérieur de la cavité (5) du corps de soutien (2) est adapté à l'extension latérale du ligament (7a, 7b) et une paroi intérieure (4a) de la cavité (5) entoure étroitement un ligament (7a, 7b) à guider sur la circonférence, de sorte que la formation d'une matrice de cicatrisation primaire est possible autour du ligament (7a, 7b) guidé dans la cavité, et
**en ce que** un ou plusieurs facteurs de croissance et/ou une ou plusieurs substances favorisant la cicatrisation peuvent être injectés dans la cavité interne (5) par des moyens d'injection (15), et
**en ce que** les première et seconde ouvertures (3a, 3b) sont disposées à des extrémités opposées du corps de soutien (2) et sont reliées à la cavité (5) du corps de soutien.

2. L'implant selon la revendication 1,
dans lequel le corps de soutien (2) est au moins en partie constitué d'un matériau élastique.

3. L'implant selon la revendication 1 ou 2,
dans lequel le corps de soutien (2) est sensiblement tubulaire ou en forme de ballonnet.

4. L'implant selon l'une des revendications 1 à 3,
dans lequel le corps de soutien (2) a une structure réticulaire, analogue à une mousse ou analogue à un gel.

5. L'implant selon l'une des revendications précédentes,
dans lequel le corps de soutien (2) est constitué d'un matériau tissé à mailles serrées.

6. L'implant selon l'une des revendications précédentes,
dans lequel le corps de soutien (2) est constitué d'un matériau résorbable par le corps humain ou animal.

7. L'implant selon l'une des revendications précédentes,
dans lequel le matériau du corps de soutien (2) comprend un ou plusieurs des matériaux suivants : collagène de type 1 ou 2, polyuréthane, acide polycyclique.

8. L'implant selon l'une des revendications précédentes,
dans lequel le corps de soutien (2) comporte au moins en partie une substance favorisant la croissance d'un ligament (7a, 7b).

9. L'implant selon la revendication 8,
dans lequel une surface intérieure (4a) de la paroi extérieure (4) du corps de soutien (2) est au moins partiellement revêtue de la substance favorisant la croissance.

10. L'implant selon la revendication 8 ou 9,
dans lequel la substance favorisant la croissance comprend une ou plusieurs des substances suivantes : cellules souches pluripotentes, fibrine, protéines matricielles extra-cellulaires, vitronectine, cellules souches de graisse autologue, facteurs de croissance, cytokines, HGF (facteur de croissance humain), interleukine-1, IL-3, IL-6, IL-11, PDGF (facteur de croissance dérivé des plaquettes), FGF (facteur de croissance des fibroblastes), EGF (facteur de croissance épidermique), VEGF (facteur de croissance endothéliale vasculaire), PRP (plasma riche en plaquettes), PRE (fibrine riche en plaquettes) et/ou angiopoiétine.

11. L'implant selon l'une des revendications précédentes, dans lequel le corps de soutien (2) comporte, au niveau des deux extrémités opposées, des moyens de fixation (6) pour la fixation et le positionnement sur un os (10, 11).
